# EUROPÄISCHE PATENTSCHRIFT

(11) **EP 2 024 489 B1**
(45) Veröffentlichungstag und Bekanntmachung des Hinweises auf die Patenterteilung: **24.06.2015**
(21) Anmeldenummer: 07724993.6
(22) Anmeldetag: 08.05.2007
(51) Int. Cl.: C12N 1/18

(54) **VERFAHREN ZUR PRODUKTION VON HEFE**
PROCESS FOR THE PRODUCTION OF YEAST
PROCÉDÉ DE PRODUCTION DE LEVURE

(30) Priorität: 12.05.2006 DE 102006022716
(43) Veröffentlichungstag der Anmeldung: 18.02.2009
(60) Teilanmeldung: 09007174.7
(73) Patentinhaber: Beta Tec Hopfenprodukte GmbH, 90482 Nürnberg (DE)
(72) Erfinder: BACZYNSKI, Lilith, 91564 Neuendettelsau (DE); BEDDIE, David, Herefordshire HR6 0BG (GB); WIRTH, Tobias, 91126 Schwabach (DE)
(74) Vertreter: Stippl, Hubert
(86) Internationale Anmeldenummer: PCT/EP2007/004070
(87) Internationale Veröffentlichungsnummer: WO 2007/131669

(56) Entgegenhaltungen:
- WO-A-00/52212
- WO-A-01/88205
- WO-A-2004/072291
- US-A- 1 477 132
- US-B1- 6 326 185

## Beschreibung

Hefen sind die weltweit wichtigste Gruppe von Mikroorganismen von kommerziellem Wert. Sie werden schon seit Jahrtausenden von Menschen verwendet.

Die Gesamtmenge der Hefeproduktion einschließlich der Hefeproduktion beim Brauen und Fermentieren und bei der Lebensmittelproduktion beläuft sich auf Millionen Tonnen pro Jahr. Die Hefeproduktion in der EU hat einen Umsatz von 800 Millionen Euro und sichert ohne Berücksichtigung von Zuliefererindustriezweigen über 8000 Arbeitsplätze.

Die aus wirtschaftlicher Sicht wichtigste Hefegattung ist *Saccharomyces cervisiae.* Es gibt jedoch zahlreiche weniger geläufige Hefearten mit Potential für technologische Anwendungen. Ein wichtiger technischer Hefestamm ist beispielsweise *Kluyveromyces lactis.* Diese Mikroben werden in Fermentern zur Herstellung von Chymosin (Rennin) in großtechnischem Maßstab herangezogen; dieses Rennin, das die aus geschlachteten Tieren gewonnene herkömmliche Form ersetzt, findet heutzutage in der Käseproduktion breite Anwendung.

*Saccharomyces*-Hefen werden im allgemeinen als unbedenklich ("GRAS") eingestuft. Sie produzieren zwei wichtige Stoffwechselprodukte - Ethanol und Kohlendioxid. Ethanol wird zur Produktion von alkoholischen Getränken, als Kraftstoff und als Lösungsmittel verwendet. Kohlendioxid wird als Triebmittel für Brotteig, zur Herstellung von kohlensäurehaltigen Getränken, als Schutzgas bei der Lebensmittelkonservierung und als Lösungsmittel für Extraktionen verwendet. Hefe selbst wird als Geschmacksverbesserer in Lebensmitteln und als Nucleotidquelle bei der Produktion von Muttermilchersatzstoffen verwendet. Hefe ist auch darüber hinaus eine wichtige Vitamin-B-Quelle in der menschlichen und tierischen Ernährung. Steriler Hefeextrakt wird bei der Kultivierung von industriellen Schimmelpilzkulturen zur Enzymproduktion oder bei der Produktion von Starterkulturen verwendet. Futterhefe wird in Vieh- und Pferdefutter zur Stabilisierung der anaeroben Umgebung im Pansen verwendet. Gentechnisch veränderte Hefe wird zur Produktion von Proteinen wie Insulin, die in der Humanmedizin Anwendung finden, verwendet.

Hefe wird stets aus einer Reinhefekultur produziert, welche durch Selektion, Zucht oder gentechnische Veränderung bezüglich Leistungsfähigkeit bei der vorgesehenen Anwendung isoliert worden ist. Bei der Produktion von reinen Hefekulturen bedient man sich des Doppelprinzips der Verwendung einer bakteriologisch einwandfreien Probe für die Anfangsinokulation und der Beibehaltung dieser Reinheit über die gesamte Hefeproduktion hinweg. Backhefe (*Saccharomyces cervisiae)* hat ihren Ursprung in obergäriger Bierhefe. Durch Selektion von bestimmten Hefestämmen und deren Eigenschaften und anschließende Vermehrung der Zellen wird eine Reinhefekultur auf einem Nährboden aus Melasse und verschiedenen Zusatzstoffen gewonnen. Backhefe ist beispielsweise durch hohe Triebkraft und einen geringen Gehalt an Glutenzersetzenden Enzymen gekennzeichnet. Während der jeweilige Hefestamm natürlich das Betriebsgeheimnis des Hefeproduzenten ist, ist der technische Ablauf der Hefevermehrung allgemein bekannt.

Zur Produktion einer großen Menge von Biomasse in Reinkultur ist in der Biotechnologie eine mehrstufige Kultivierung üblich. Bei einer einstufigen Kultivierung, bei der ein großes Substratvolumen mit einer kleinen Menge der betreffenden Mikroorganismen inokuliert wird, würde eine lange Zeit für die ausreichende Biomassevermehrung benötigt. Das hätte einige Nachteile: Eine längere Lagerperiode, in der die Hefe eine optimale Umgebung für das Hefewachstum schafft (verringertes Redoxpotential, Konzentration von Wachstumsstimulatoren), hohes Risiko der Kontamination mit unerwünschten Organismen und wirtschaftliche Ineffizienz.

Hefe verwertet ein breites Spektrum von Kohlenhydraten und Zuckern. Derzeit ist jedoch keine Hefeart bekannt, die in der Lage ist, alle verfügbaren Zucker zu verwerten. Der obergärige Stamm *Saccharomyces cerevisiae* verwertet charakteristischerweise Glucose, Fructose, Mannose, Galactose, Saccharose, Maltose, Maltotriose und Raffinose. *Saccharomyces cerevisiae* und verwandte Stämme können jedoch keine C5-Zucker, wie Ribose, Xylose, Arabinose oder Cellobiose, verwerten.

Das gebräuchlichste Substrat für die Kultivierung von Hefe ist eine wäßrige Lösung von 8-10% Melasse. Melasse enthält etwa 50 % Zucker. Die Melasse wird zur Abtrennung jeglichen Schlamms geklärt und dann mit Hochdruckdampf sterilisiert. Danach wird sie mit Wasser verdünnt und in Pufferbehältern aufbewahrt, bis sie für den Fermentationsprozeß gebraucht wird. Die Lösung wird mit Hilfe anorganischer Säuren auf einen pH-Wert von etwa 4,5-5,0 gebracht und mit Mineralien und Vitaminen der B-Gruppe, die für die Hefeproduktion benötigt werden, angereichert. Zum Nährstoff- und Mineralienbedarf gehören Stickstoff, Kalium, Phosphat, Magnesium und Calcium mit Spuren von Eisen, Zink, Kupfer, Mangan und Molybdän. Normalerweise wird dem Substrat Stickstoff durch Zusatz von Ammoniumsalzen, wäßrigem Ammoniak oder wasserfreiem Ammoniak zugeführt. Phosphate und Magnesium werden in Form von Phosphorsäure oder Phosphatsalzen und Magnesiumsalzen zugegeben. Für das Hefewachstum sind auch Vitamine erforderlich (Biotin, Inosit, Pantothensäure und Thiamin).

Die Hefekulturen werden belüftet, um eine möglichst hohe Biomasse-Produktion zu erzielen. Hefen sind hochentwickelte einzellige Pilze. Sie sind fakultativ anaerob: In Gegenwart von Luft produzieren sie aus Zucker und Sauerstoff Kohlendioxid und Wasser. Dieser Stoffwechselprozeß wird als Atmung bezeichnet und erzeugt eine Menge Energie (ATP). Bei der Atmung wird Glucose vollständig oxidiert und somit die gesamte darin enthaltene biochemische Energie freigesetzt.

Glucose + Sauerstoff → Kohlendioxid + Wasser + Energie (38 ATP)

Diese Energie wird von Hefe zur Aufrechterhaltung von Lebensfunktionen und zur Synthese von Biomasse, d.h. Hefewachstum, verwendet. Neben Sauerstoff und Zucker sind für die Produktion von Biomasse noch einige andere Substanzen erforderlich, insbesondere Stickstoff.

In Abwesenheit von Sauerstoff kann Hefe trotzdem noch aus Zucker Energie zur Aufrechterhaltung der Lebensfunktionen produzieren. Der anaerobe Stoffwechsel in Hefe wurde von Louis Pasteur als Fermentation definiert. Bei der Fermentation werden aus Zucker Kohlendioxid und Ethanol produziert. Die Oxidation der Glucose ist unvollständig. Ethanol enthält eine Menge Energie, so daß nur ein kleiner Teil der in Glucose enthaltenen biochemischen Energie freigesetzt wird:

Glucose → Kohlendioxid + Ethanol + Energie (2 ATP)

Demzufolge ist die Hefezellvermehrung unter anaeroben Bedingungen ziemlich stark eingeschränkt. Die technische Hefeproduktion basiert auf dem aeroben Prozeß. Zur Erzeugung von aeroben Bedingungen wird Luft durch die Lösung geblasen, in welcher die Hefe herangezogen wird.

Das sich dem Hefehersteller stellende Problem ist jedoch nicht so einfach wie die simple Zufuhr von Luft während des Fermentationsprozesses. Wenn die Zuckerkonzentration im Wachstumsmediumeinegeringe Menge übersteigt, wird die Hefe auch bei ausreichender oder gar reichlicher Luftzufuhr etwas Alkohol produzieren (Crabtree-Effekt). Dieses Problem kann durch langsame Zugabe der Zuckerlösung zu der Hefe über den gesamten Fermentationsprozeß hinweg behoben werden. Die Zugaberate der Zuckerlösung muß so gewählt sein, daß die Hefe den Zucker so schnell verbraucht, daß die Zuckerkonzentration zu einem beliebigen Zeitpunkt praktisch null ist. Diese Art von Fermentation wird als Fed-Batch-Fermentation bezeichnet.

Bei der Herstellung von Backhefe wird die Hefe in mehreren Stufen kultiviert. Beispielsweise könnte Hefe aus einer Reagenzglaskultur in Substrat mit 50 ml, dann 1 Liter, 10 Liter, 40 Liter, 400 Liter, 4 m³, 10 m³ und schließlich 200 m³ überführt werden. Die Anfangsstufe des Hefewachstums erfolgt im Labor. Ein Teil der Reinhefekultur wird in einem sterilisierten Kolben mit Melassewürze vermischt, indem die Hefe 2 bis 4 Tage anwachsen kann. Der gesamte Inhalt dieses Kolbens wird dann zur Beimpfung des ersten Fermenters in der Reinkulturstufe verwendet. Reinkulturfermentationen sind diskontinuierliche Fermentationen, in denen die Hefevermehrung 13 bis 24 Stunden beträgt. In dieser Verfahrensstufe werden in der Regel 1 bis 2 Fermenter verwendet.

Bis auf die Tatsache dass Vorkehrungen für sterile Belüftung und aseptische Überführung in die nächste Stufe fehlen, handelt es sich bei den Reinkulturfermentationen im Grunde genommen um eine Fortsetzung der Kolbenfermentation. aufweisen. Diese frühen Stufen werden als Set-Batch-Fermentationen durchgeführt. Dabei werden alle Wachstumsmedien und Nährstoffe vor der Inokulation in den Behälter eingetragen.

Bei der nächsten Fermentationsstufe handelt es sich um eine Stammkulturfermentation. Der Inhalt des Zwischenfermenters wird in den Stammfermenter gepumpt, welcher auf kontiunuierliche Belüftung ausgelegt ist. Diese Stufe wird als Stellhefefermentationbezeichnet, da die Hefe nach abgeschlossener Fermentation durch Zentrifugieren vom größten Teil der Fermenterflüssigkeit abgetrennt wird, was eine Stammkultur für die Beimpfung der nächsten Stufe ergibt. Die Belüftung ist kräftig, und Melasse und andere Nährstoffe werden schrittweise zugeführt. Die Flüssigkeit aus diesem Fermenter wird in der Regel in mehrere Teile zum Anstellen der abschließenden Versandhefekultivierung geteilt. Alternativ dazu kann die Hefe durch Zentrifugieren abgetrennt und vor der Verwendung bei den abschließenden Versandhefekultivierungen einige Tage aufbewahrt werden.

Die Einführung des Fed-Batch-Prozesses in die Hefeproduktion kennzeichnet den Beginn der modernen Hefeindustrie. Die Fed-Batch-Produktion wurde zuerst in Deutschland im Jahre 1915 eingeführt, und die moderne Hefeproduktion wird immer noch als Fed-Batch-Verfahren durchgeführt. Bei einer Fed-Batch-Fermentation werden Melasse, Phosphorsäure, Ammoniak und Mineralien der Hefe mit einer kontrollierten Rate zugeführt. Diese Rate ist darauf ausgerichtet, der Hefe gerade so viel Zucker und Nährstoffe zuzuführen, daß die Vermehrung maximiert und die Produktion von Alkohol verhindert wird.

Zu Beginn jeder Charge wird zusammen mit der Stellhefe eine ziemlich beträchtliche Menge an Prozeßwasser in den Fermenter überführt. Die optimale Menge an zuzuführendem Zucker pro Gramm Hefe und pro Stunde ist festgelegt. Melasse wird dem Fermenter in einer Rate zugeführt, die der vorbestimmten Menge an zuzuführendem Zucker entspricht.

Bei der Produktion von Backhefe ist eine Grundzufuhr von 16-18 g/l gebräuchlich. Stickstoff und Phosphor können entweder mit einer bestimmten Rate zugeführt oder vor der Melassezufuhr dem Prozeßwasser beigemischt werden. Die Mengen werden durch das Gewichtsverhältnis von Zucker, Stickstoff und Phosphor bestimmt. Nach 8-16 h Kultivierung ist die Hefekultur gereift. Für die Hefeproduktion werden 2,5 g Stickstoff und 5,0 g Phosphor bzw. 0,3 g Stickstoff und 0,5 g Phopshor pro 100 g Zucker benötigt.

Die Kultivierungstemperatur liegt üblicherweise zwischen 25 und 35°C und der pH-Wert wird durch die Mengen an zugesetztem Stickstoff bestimmt. Die Stellhefeproduktion wird bei einem pH-Wert zwischen 4,5 und 5,0 durchgeführt., die Versandhefeproduktion bei einem pH-Wert zwischen 5,0 und 7,0.

Die abschließende Vesandhefefermentation hat den höchsten Belüftungsgrad, und Melasse und andere Nährstoffe werden schrittweise zugeführt. Bei den abschließenden Vesandhefefermentationen ist eine beträchtliche Luftzufuhr erforderlich, so daß diese Behälter oft zeitlich versetzt gestartet werden, um die Größe der Luftverdichter zu verringern. Die Dauer der abschließenden Fermentationsstufen liegt im Bereich von 11 bis 15 Stunden. Nach der Zufuhr der gesamten benötigten Melasse zum Fermenter wird die Flüssigkeit noch 0,5 bis 1,5 Stunden belüftet, um eine weitere Reifung der Hefe zu gestatten, was die Hefe stabiler gegenüber dem Kühltransport macht. Außerdem gewährleistet diese Methode, daß kleine Ethanolmengen durch Veratmung entfernt werden.

Am Ende der Fermentation wird die Fermenterbrühe durch Zentrifugen abgetrennt, mit Wasser gewaschen und erneut zentrifugiert, was eine Hefemilch mit einer Feststoffkonzentration von ungefähr 18% ergibt. Die Hefemilch wird .auf etwa 8°C abgekühlt und in einem separaten, gekühlten Hefemilchbehälter aus rostfreiem Edelstahl aufbewahrt. Die Hefemilch kann direkt in Tanklastzüge geladen und zu Kunden mit einem entsprechenden Hefemilchhandhabungssystem befördert werden. Vor 1825 war Hefe nur als Hefemilch erhältlich. Im Jahre 1825 wurde von Tebbenhof Preßhefe eingeführt. Mit der Entwicklung von Großhandelsbäckereien für die Massenlieferung wurde die Hefemilchherstellung wieder populär.

Alternativ dazu kann man die Hefe zentrifugieren und die Hefefeststoffe mit Hilfe einer Filterpresse oder eines Vakuumrotationsfilters weiter aufkonzentrieren. Eine Filterpresse ergibt einen Filterkuchen mit 27 bis 32 Prozent Feststoffen. Ein Vakuumrotationsfilter ergibt Filterkuchen mit ungefähr 33 Prozent Feststoffen.

Der Filterkuchen wird dann in Mischern mit kleinen Mengen Wasser, Emulgatoren und Verschnittölen zum Endprodukt vermischt. Bei der Preßhefeproduktion werden Emulgatoren zugegeben, um der Hefe ein weißes, cremiges Aussehen zu verleihen und die Bildung von Wasserflecken auf dem Hefekuchen zu inhibieren. Eine kleine Menge Öl, üblicherweise Sojabohnen- oder Baumwollsaatöl, wird zugegeben, um die Extrusion der Hefe durch Düsen zu Endlosbändern von Hefekuchen zu unterstützen. Die Bänder werden geschnitten und die Hefekuchen abgepackt und auf eine Temperatur von weniger als 8°C abgekühlt, wonach sie für den Kühllastertransport bereit sind. Zur Produktion von einer Tonne Preßhefe mit 27% Trockensubstanz (Y27) werden 100 kg bis 250 kg Stellhefe und eine Tonne Melasse benötigt.

Bei der Herstellung von aktiver Trockenhefe wird Preßhefe zu Zylindern geformt, die dann im Wirbelschichttrockner getrocknet werden. Aktive Trockenhefe kann bei Raumtemperatur aufbewahrt werden.

Zur Herstellung von Bio-Hefe wird das Melassesubstrat durch Stärkesubstrate ersetzt. Weizenstärke, Maisstärke oder Kartoffelstärke werden mit technischen Enzymen zu einer Zuckerlösung verzuckert. Bio-Backhefe produziert weniger Kohlendioxid, da das Endprodukt etwa 30% Stärke enthält.

Fed-Batch-Fermentationen sind nicht vollkommen steril. Die Verwendung von Druckbehältern zur Gewährleistung der Sterilität der in diesen Fermentern benötigten großen Luftvolumina oder zur Erzielung von sterilen Bedingungen bei allen Transferprozessen durch die zahlreichen Leitungen, Pumpen und Zentrifugen ist nicht wirtschaftlich. Zur Gewährleistung von möglichst aseptischen Bedingungen werden die Einrichtungen ausgiebig gereinigt, Rohre und Behälter mit Wasserdampf beaufschlagt und die Luft filtriert. Es ist jedoch nahezu unmöglich, eine gewisse Form von mikrobieller Verunreinigung zu vermeiden, und es ist über die Anwesenheit von Bakterien wie *Leuconostoc, Pediococcus, Aerococcus, Bacillus, Lactococcus* und *E.coli* berichtet worden. Die Fed-Batch-Stufen werden über einen begrenzten Zeitraum (10-20 Stunden) kultiviert. Durch den vergleichsweise kurzen Kultivierungszeitraum und ein großes Hefeinokulum sollen Fremdorganismen darin gehindert werden, sich zu einer sichtbaren Infektion auszuwachsen. Gegenwärtig scheint die Infektionsbekämpfung auf physikalische Methoden beschränkt zu sein, wie beispielsweise die Verwendung von Waschseparatoren zur Entfernung von möglichst vielen Bakterien zusammen mit der Hefewürze durch den Dichteunterschied bei der Ernte der Hefezellen.

Zuweilen wird als Präventivmaßnahme die Senkung des pH-Werts vorgeschlagen, jedoch kann das optimale Hefewachstum nur bei einem pH-Wert um 6 erreicht werden. Manchmal wird Kochsalz verwendet, um die Konservierung der Preßhefe über einen längeren Zeitraum zu unterstützen.

Die Infektionsbekämpfung bei der Hefeproduktion ist für die Gewährleistung der Reinheit der Hefekultur unerläßlich. Bakterielle Verunreinigungen sind besonders störend, wenn Hefe in Fermentationen verwendet wird, wie in der Bier- oder Ethanolproduktion. In diesem Fall könnte die Hefe als Infektionsquelle für den Fermentationsprozeß dienen. Den Hefeherstellern ist durchaus bewußt, daß die Zahl der bakteriellen Verunreinigungen pro Gramm Hefemilch, Preßhefe oder aktiver Trockenhefe ein entscheidender Faktor für die Hefequalität ist.

Die vorliegende Erfindung stellt ein Verfahren zur Bekämpfung der bakteriellen Verunreinigung während der Hefeproduktion und Hefelagerung bereit.

Die US-PS 6,326,185 B1 bezeichnet ein Verfahren zur Dekontamination von in der Fermentation verwendeter Bierhefe, damit diese für nachfolgende Fermentationen verwendet werden kann. Die Hefe wird mit Tetrahydroiso-α-säuren bis zu einer Endkonzentration von 40 ppm in Berührung gebracht, und gleichzeitig wird der pH-Wert der Mischung auf etwa 2,0 bis 2,6 eingestellt. Dann wird die Mischung über einen speziellen Zeitraum bei einer speziellen Temperatur gehalten.

Die US-PS 1,477,132 betrifft eine Hefezusammensetzung, im einzelnen eine Hefezusammensetzung für die Reisfermentation, und ein Herstellungsverfahren dafür. Gekochter Reis wird mit Hopfensaft versetzt. Der Reis wird dann so mit Hefe behandelt, daß die Hefe wachsen kann. Der Zusatz von Hopfensäure dient zur Vermeidung von Oxidation, Zersetzung und anderen giftigen Stoffwechselprozessen durch Bakterien.

Die WO 2004/072291 A2 betrifft die Verwendung von Hopfensäuren bei der Bioethanolproduktion zur Bekämpfung von Mikroorganismen. In dieser Anmeldung wird nur allgemein erwähnt, daß das Verfahren zur Bekämpfung von Mikroorganismen durch Verwendung von Hopfensäure auch bei der Vermehrung von Hefe angewandt werden kann.

Aufgabe der vorliegenden Erfindung ist die Bereitstellung eines Verfahrens zur technischen Produktion von Hefe, bei dem die Aktivität von Mikroorganismen verhältnismäßig kostengünstig vermieden oder zumindest verringert werden kann.

Eine weitere Aufgabe der vorliegenden Erfindung besteht in der Bereitstellung eines Verfahrens zur technischen Produktion von Hefe, bei dem physikalische Verfahren zur Verringerung der Aktivität von unerwünschten Mikroorganismen in Hefe vermieden oder zumindest vereinfacht werden können.

Eine weitere Aufgabe der vorliegenden Erfindung besteht in der Bereitstellung eines Verfahrens zur technischen Produktion von Hefe, bei dem bauliche Maßnahmen, z.B. Druckbehälter in der Stufe der Fed-Batch-Fermentierung von Hefe vermieden oder zumindest vereinfacht werden können.

Eine weitere Aufgabe der vorliegenden Erfindung besteht in der Bereitstellung eines Verfahrens zur technischen Produktion von Hefe, bei dem die erforderliche Intensität der Reinigung der Einrichtungen verringert werden kann.

Eine weitere Aufgabe der vorliegenden Erfindung besteht in der Bereitstellung eines Verfahrens zur technischen Produktion von Hefe, insbesondere Versandhefe, bei dem die Verwendung von chemischen Antischaummitteln verringert oder vermieden werden kann.

### KURZE DARSTELLUNG DER ERFINDUNG

Erfindungsgemäß bringt man Hefe mit einem natürlichen oder einem aus einem Naturstoff abgeleiteten Verarbeitungshilfsmittel in einer ausreichenden Menge zur Inhibierung oder zumindest Verringerung der Aktivität von Mikroorganismen bei der technischen Produktion von Stellhefe und/oder Versandhefe in Berührung, wobei das natürliche oder das aus einem Naturstoff abgeleitete Verarbeitungshilfsmittel unter mindestens einem Mitglied der Gruppe umfassend Hopfensäuren oder einem Derivat davon ausgewählt ist. Dadurch können andere teure Maßnahmen zur Vermeidung oder zumindest Verringerung der Aktivität von unerwünschten Mikroorganismen effektiv vermieden werden.

Vorzugsweise gibt man das natürliche oder aus einem Naturstoff abgeleitete Verarbeitungshilfsmittel vor der Zugabe der Hefekultur zu dem Kohlenhydratsubstrat für die Kultivierung.

Vorzugsweise gibt man das natürliche oder aus einem Naturstoff abgeleitete Verarbeitungshilfsmittel vor einer Wärmebehandlung des Substrats zu. Vorzugsweise wird das natürliche oder aus einem Naturstoff abgeleitete Verarbeitungshilfsmittel kontinuierlich zugegeben.

Zusätzlich oder alternativ dazu gibt man das natürliche oder aus einem Naturstoff abgeleitete Verarbeitungshilfsmittel zu dem Schritt der Produktion von Stellhefe. Da die Stellhefe vor der Überführung in den Produktionsschritt von Versandhefe gewaschen wird, kann Hopfensäure und insbesondere Tetrahydroiso-α-säure mit bitterem Geschmack überraschenderweise ohne negative Auswirkungen auf den Geschmack des fertigen Hefeprodukts verwendet werden.

Zusätzlich oder alternativ dazu gibt man das natürliche oder aus einem Naturstoff abgeleitete Verarbeitungshilfsmittel zu dem Produktionsschritt von Versandhefe.

Je nach dem pH-Wert des jeweiligen Produktionsschritts werden besondere Säuren oder Derivate davon bereitgestellt, um die beste Wirkung zu erzielen.

Vorzugsweise wird das natürliche oder das aus einem Naturstoff abgeleitete Verarbeitungshilfsmittel diskontinuierlich zugegeben, mit anderen Worten als Schockdosierung, vorzugsweise zu Beginn des Produktionsschritts. Infolgedessen ist die Konzentration des natürlichen oder des aus einem Naturstoff abgeleiteten Verarbeitungshilfsmittels zu Beginn des Produktionsschritts höher und nimmt im Lauf der Zeit ab. Wenn Mikroorganismen in den Produktionsschritt durch kontaminierten Nährstoff oder durch kontaminierte Einrichtungen eingeschleppt werden, die somit den Hefeproduktionsprozeß beeinträchtigen, wird die Aktivität dieser Mikroorganismen von Anfang an verhindert, so daß der Hefeproduktionsprozeß weder durch anfängliche Kontamination noch durch später eingeschleppte Kontamination nachteilig beeinflußt wird. Da das natürliche oder aus einem Naturstoff abgeleitete Verarbeitungshilfsmittel als Schockdosierung zu Beginn des Produktionsschritts zugegeben wird, wird außerdem seine Konzentration im Lauf der Zeit des Hefeproduktionsprozesses verdünnt, wodurch der bittere Geschmack von Hopfensäure verringert oder vermieden wird.

Das trifft insbesondere dann zu, wenn es sich bei dem Produktionsverfahren von Stellhefe um ein Fed-Batch-Verfahren handelt. Die Konzentration des natürlichen oder aus einem Naturstoff abgeleiteten Verarbeitungshilfsmittels nimmt im Lauf des Fed-Batch-Verfahrens ab.

Vorzugsweise wählt man das natürliche oder aus einem Naturstoff abgeleitete Verarbeitungshilfsmittel unter mindestens einem Mitglied der Gruppe von Hopfensäuren, die alpha-Säuren, beta-Säuren, rho-Iso-α-säuren, Iso-α-säuren, Hexahydroiso-α-säuren, Tetrahydroiso-α-säuren und Hexahydro-beta-säuren umfaßt, aus.

Vorzugsweise können Tetrahydroiso-α-säuren oder beta-Säuren alleine oder in Kombination als natürliches oder aus einem Naturstoff abgeleitetes Verarbeitungshilfsmittel im Lauf des Verfahrensschritts der Herstellung von Stellhefe zugegeben werden. Tetrahydroiso-α-säuren sind bei dem im allgemeinen bei der Stellhefeproduktion anzutreffenden pH-Wert von 4-5 die wirksamste Hopfenverbindung. Daher werden Tetrahydroiso-α-säuren am zweckmäßigsten bei diesem Verfahrensschritt zugegeben.

Die Ausgangskonzentration an Tetrahydroiso-α-säuren liegt im Bereich von 1-1000 ppm, vorzugsweise 10-100 ppm, vorzugsweise 20-60 ppm und ganz besonders bevorzugt 15-30 ppm. Durch diese Konzentration wird die bakterizide Wirkung bereitgestellt. Andererseits wird die Hopfensäure durch das gesamte Produktionsverfahren so stark verdünnt, daß sie den Geschmack der Hefe nicht nachteilig beeinflußt.

Die Endkonzentration an Tetrahydroiso-α-säuren in dem behandelten Behälter liegt in einem Bereich von 5-20 ppm, vorzugsweise 3-15 ppm und ganz besonders bevorzugt 2-10 ppm, was zur Inhibierung des Bakterienwachstums ausreicht.

Vorzugsweise gibt man beta-Säuren als natürliches oder aus einem Naturstoff abgeleitetes Verarbeitungshilfsmittel im Produktionsschritt von Versandhefe zu. Es wurde ermittelt, daß beta-Säuren dabei helfen, die durch elektrochemische Wechselwirkung mit Bakterien verursachte Ausflockung von Hefezellen zu verhindern. Außerdem sind beta-Säuren auch für den Produktionsschritt von Versandhefe am besten geeignet, da sie am besten bei pH 5-7, dem üblichen pH-Wert im Schritt der Produktion von Versandhefe, wirken. Des weiteren wurde ermittelt, daß beta-Säuren im Produktionsschritt von Versandhefe eine Antischaumwirkung bereitstellen. Infolgedessen hilft die Verwendung von beta-Säuren, die als natürliches Verarbeitungshilfsmittel im Produktionsschritt von Versandhefe zugegeben werden, bei der Verringerung oder sogar Vermeidung der Verwendung von chemischen Antischaummitteln.

Vorzugsweise liegt die Ausgangskonzentration an beta-Säuren im Bereich von 1-1000 ppm, vorzugsweise 10-100 ppm und ganz besonders bevorzugt 40-60 ppm.

Vorzugsweise liegt die Endkonzentration an beta-Säuren im behandelten Behälter im Bereich von 8-300 ppm, vorzugsweise 3-30 ppm und ganz besonders bevorzugt 1-12 ppm.

### BESCHREIBUNG DER ERFINDUNG

Im folgenden werden Ausführungsformen des erfindungsgemäßen Verfahrens unter Bezugnahme auf die Figuren beschrieben. Diese Figuren zeigen:
- Fig. 1: zeigt ein Beispiel für die Produktion von Backhefe auf Melassesubstrat;
- Fig. 2: betrifft ein Diagramm, das die Inhibierung des Bakterienwachstums in MRS-Bouillon bei pH 5 mit Tetrahydroiso-α-säuren und beta-Säuren zeigt.

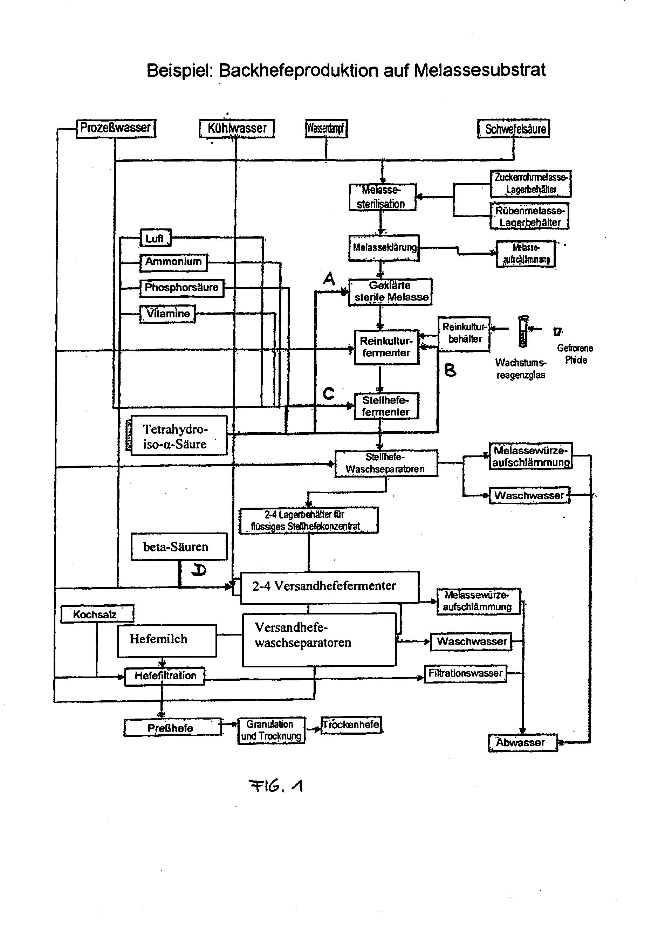

Figur 1 zeigt eine schematische Illustration (Fließbild) des Herstellungsverfahrens für Backhefe. Eine Mischung von Zuckerrohrmelasse und Rübenmelasse wird zunächst durch Wärmebehandlung mit Hochdruckdampf sterilisiert, und der pH-Wert wird durch Ansäuern mit Schwefelsäure eingestellt. Die Melasse wird dann zur Entfernung jeglichen Schlamms geklärt. Geklärte, sterile Melasse wird als sogenanntes Kohlenhydratsubstrat für die Produktion von Hefe-Biomasse verwendet.

Gemäß einer ersten Ausführungsform der vorliegenden Erfindung werden Hopfensäuren, insbesondere Tetrahydroiso-α-säuren, zu der geklärten sterilen Melasse gegeben (siehe Linie A in Figur 1), um jegliche Aktivität von grampositiven Bakterien zu verhindern, insbesondere bei den anschließend folgenden Prozessen. Bei dem Substrat handelt es sich im allgemeinen um eine wäßrige Lösung von 8-10% Melasse. Die Melasse enthält ungefähr 50% Zucker.

Die geklärte sterile Melasse wird in den Reinkulturfermenter eingetragen, in dem die Melasse mit Prozeßwasser und Reinkultur versetzt wird.

Gemäß einer weiteren Ausführungsform der vorliegenden Erfindung werden Hopfensäuren, insbesondere Tetrahydroiso-α-säuren, in den Reinkulturfermenter gegeben (siehe Linie B in Figur 1), um die Aktivität von unerwünschten Mikroorganismen zu verhindern oder zumindest zu verringern.

Danach wird die Mischung aus der wäßrigen Melasselösung und der Reinhefekultur in einen Stellhefefermenter überführt. Im Stellhefefermenter wird die Lösung auf einen pH-Wert von etwa 4,0-5,0 eingestellt und mit Mineralien und Vitaminen, speziell der B-Gruppe, die für ein ordentliches Hefewachstum notwendig sind, angereichert. Zum Nährstoff- und Mineralienbedarf gehören Stickstoff, Kalium, Phosphat, Magnesium und Calcium mit Spuren von Eisen, Zink, Kupfer, Mangan und Molybdän. In der Regel wird Stickstoff durch Zusatz von Ammoniumsalzen, wäßrigem Ammoniak oder wasserfreiem Ammoniak zum Einsatzstoff zugeführt. Phosphate und Magnesium werden in Form von Phosphorsäure oder Phosphatsalzen und Magnesiumsalzen zugegeben.

Außerdem werden die Hefekulturen in der Stellhefeproduktion belüftet, um eine maximale Biomasseproduktion zu erhalten.

Gemäß einer weiteren Ausführungsform der vorliegenden Erfindung werden Hopfensäuren in den Stellhefefermenter eingetragen (siehe Linie C in Figur 1), um die Aktivität von unerwünschten Mikroorganismen zu inhibieren oder zumindest zu verringern.

Die Zugabe wird vorzugsweise als Schockdosierung zu Beginn des Stellhefefermentationsverfahrens durchgeführt.

Die Endkonzentration der Hopfensäuren, insbesondere Tetrahydroiso-α-säuren, liegt zwischen 5 und 10 ppm. Es wurde ermittelt, daß Tetrahydroiso-α-säuren bei der Unterdrückung oder zumindest Verringerung der Aktivität von Mikroorganismen im Produktionsschritt von Stellhefe am effektivsten sind. Daher reichen schon geringe Mengen an Tetrahydroiso-α-säuren aus, um das gewünschte Ergebnis zu erhalten. Das Stellhefefermentationsverfahren wird als Fed-Batch-Verfahren realisiert, bei dem der Hefe Melasse, Phosphorsäure, Ammoniak und Mineralien mit einer kontrollierten Rate zugeführt werden. Somit sinkt die Anfangskonzentration an Hopfensäuren im Lauf des Fed-Batch-Produktionsverfahrens. Dies führt dazu, daß die Hopfensäurekonzentration im Fermenter abnimmt und jegliche durch die Hopfensäure bedingte Bitterkeit des Endprodukts nach dem Waschen überraschenderweise vermieden wird.

Danach wird die Stellhefe in Stellhefewaschseparatoren gewaschen, in denen Hopfensäuren, d.h. Tetrahydroiso-α-säuren, teilweise entfernt werden und somit zusätzlich jegliche Bitterkeit im Endprodukt vermieden wird.

Die Stellhefe wird dann aufbewahrt und für die Produktion von Versandhefe in Versandhefefermentern vorbereitet. Im Schritt der Produktion von Versandhefe werden gemäß einer weiteren Ausführungsform der vorliegenden Erfindung Hopfensäuren, insbesondere beta-Säuren, zugegeben (siehe Linie D in Figur 1). Da der pH-Wert in der Produktionsstufe von Versandhefe im Bereich von 5-7 liegt, sind beta-Säuren am effektivsten. Die Anwendung von beta-Säuren im Versandhefe-produktionsverfahren stellt eine Antischaumwirkung bereit. Infolgedessen kann die Verwendung von chemischen Antischaummitteln verringert oder gar vermieden werden.

Des weiteren hat die Zugabe von beta-Säuren den vorteilhaften Effekt, daß die häufig durch elektrochemische Wechselwirkung mit Bakterienhefezellen verursachte Ausflockung von Hefezellen vermieden werden kann, da beta-Säuren das Haften derartiger unerwünschter Mikroorganismen auf der Oberfläche der Hefezellen verhindern.

Nach Beendigung des Versandhefefermentationsverfahrens wird die Versandhefe gewaschen und zu Hefemilch, Preßhefe oder letztendlich zu Trockenhefe weiterverarbeitet.

Gemäß einer anderen, nicht in Figur 1 illustrierten Ausführungsform der vorliegenden Erfindung können natürliche oder aus einem Naturstoff abgeleitete Verarbeitungshilfsmittel gegen jegliche Aktivität von vielleicht auch durch Reinfektion bedingten unerwünschten Mikroorganismen auch zur Hefemilch gegeben werden mit dem Ziel, die Haltbarkeit des Versandhefeprodukts zu verlängern.

Es versteht sich, daß die vorliegende Erfindung gemäß der vorliegenden Erfindung Zugaben von Hopfensäuren in den Schritten A, B, C und D in Kombination (A, B, C und D), als Einzelmaßnahmen (z.B. nur C) oder in Form einer Teilkombination (z.B. C und D) umfaßt.

Zusätzlich zu Hopfensäure können Kolophonium und/oder Myristinsäure als weiteres, natürliches oder aus einem Naturstoff abgeleitetes Verarbeitungshilfsmittel im Produktionsschritt von Versandhefe zugegeben werden (siehe Linie D in Figur 1).

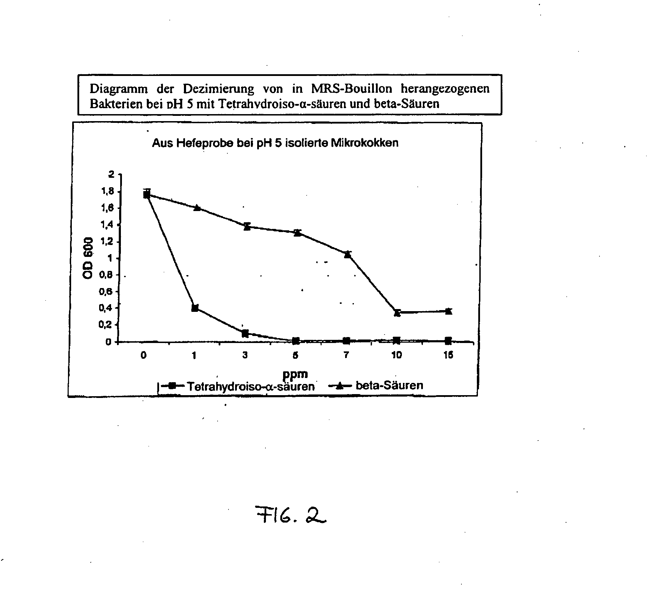

Aus Figur 2 ist die Hemmwirkung von Tetrahydroiso-α-säuren in einer Konzentration von etwa 5 ppm auf die Aktivität von Bakterien ersichtlich. Es wurde gefunden, daß beta-Säuren im Vergleich zu Tetrahydroiso-α-säuren bei niedrigerem pH-Wert weniger effektiv werden, woraus hervorgeht, daß zur Erzielung einer ähnlichen Effektivität etwa die dreifache Menge an beta-Säure im Vergleich zu Tetrahydroiso-α-säure angewandt werden muß.

## Patentansprüche

1. Verbessertes Verfahren zur Produktion von Hefe, insbesondere Backhefe, bei dem man die Hefe mit einem natürlichen oder einem aus einem Naturstoff abgeleiteten Verarbeitungshilfsmittel in einer zur Unterdrückung oder zumindest Verringerung der Aktivität von unerwünschten Mikroorganismen bei der technischen Produktion von Stellhefe und/oder Versandhefe ausreichenden Menge in Berührung bringt, wobei das natürliche oder das aus einem Naturstoff abgeleitete Verarbeitungshilfsmittel unter mindestens einem Mitglied der Gruppe umfassend Hopfensäuren oder einem Derivat davon auswählt ist.

2. Verfahren nach Anspruch 1 oder 2, bei dem man das natürliche oder das aus einem Naturstoff abgeleitete Verarbeitungshilfsmittel vor der Zugabe der Hefekultur zu einer Kohlenhydratquelle für die Hefekultivierung gibt.

3. Verfahren nach Anspruch 2, bei dem man das natürliche oder das aus einem Naturstoff abgeleitete Verarbeitungshilfsmittel vor oder nach der Wärmebehandlung des Kohlenhydratsubstrats für die Hefevermehrung zugibt.

4. Verfahren nach Anspruch 2 oder 3, bei dem man das natürliche oder das aus einem Naturstoff abgeleitete Verarbeitungshilfsmittel kontinuierlich zugibt.

5. Verfahren nach einem der vorhergehenden Ansprüche, bei dem man das natürliche oder das aus einem Naturstoff abgeleitete Verarbeitungshilfsmittel zu dem Produktionsschritt von Stellhefe gibt.

6. Verfahren nach einem der vorhergehenden Ansprüche, bei dem man das natürliche oder das aus einem Naturstoff abgeleitete Verarbeitungshilfsmittel zu dem Produktionsschritt von Versandhefe gibt.

7. Verfahren nach einem der Ansprüche 5 oder 6, bei dem man das natürliche oder das aus einem Naturstoff abgeleitete Verarbeitungshilfsmittel diskontinuierlich zugibt, vorzugsweise zu Beginn des jeweiligen Produktionsschritts.

8. Verfahren nach Anspruch 5, bei dem es sich bei dem Produktionsverfahren für Stellhefe um ein Fed-Batch-Verfahren handelt und die Konzentration des natürlichen oder des aus einem Naturstoff abgeleiteten Verarbeitungshilfsmittels im Lauf des Fed-Batch-Verfahrens abnimmt.

9. Verfahren nach Anspruch 5, bei dem man das natürliche oder das aus einem Naturstoff abgeleitete Verarbeitungshilfsmittel zum Stellhefe-Waschmedium gibt.

10. Verfahren nach einem der vorhergehenden Ansprüche, bei dem man das natürliche oder das aus einem Naturstoff abgeleitete Verarbeitungshilfsmittel unter mindestens einem Mitglied der Gruppe von Hopfensäuren, die alpha-Säuren, beta-Säuren, rho-Iso-α-säuren, Iso-α-säuren, Hexahydroiso-α-säuren, Tetrahydroiso-α-säuren und Hexahydro-beta-säuren umfaßt, auswählt.

11. Verfahren nach Anspruch 10, bei dem man die Tetrahydroiso-α-säuren oder beta-Säuren als natürliches oder aus einem Naturstoff abgeleitetes Verarbeitungshilfsmittel im Schritt der Produktion von Stellhefe zugibt.

12. Verfahren nach Anspruch 11, bei dem die Endkonzentration an Tetrahydroiso-α-säuren vorzugsweise im Bereich von 5-20 ppm, vorzugsweise 3-15 ppm und ganz besonders bevorzugt 2-10 ppm liegt.

13. Verfahren nach Anspruch 6, bei dem man beta-Säuren als natürliches Verarbeitungshilfsmittel bei dem Verfahren zur Produktion von Versandhefe zugibt.

14. Verfahren nach Anspruch 13, bei dem die Endkonzentration an beta-Säuren vorzugsweise im Bereich von 8-300 ppm, vorzugsweise 3-30 ppm und ganz besonders bevorzugt 1-12 ppm liegt.

## Claims

1. Improved method for the production of yeast, in particular baker's yeast, comprising the step of contacting the yeast with a natural processing aid or a processing aid derived from a natural substance in an amount sufficient to inhibit or at least reduce the activity of undesired microorganisms in the step of the industrial production of seed yeast and/or commercial yeast, wherein the natural processing aid or the processing aid derived from a natural substance is selected from at least one of the group comprising hop acids or a derivative thereof.

2. Method according to Claim 1 or 2, wherein the natural processing aid or the processing aid derived from a natural substance is added to a carbohydrate source for yeast cultivation before the yeast culture is added.

3. Method according to Claim 2, wherein the natural processing aid or the processing aid derived from a natural substance is added before or after the heat treatment of the carbohydrate substrate for yeast culture.

4. Method according to Claim 2 or 3, wherein the natural processing aid or the processing aid derived from a natural substance is added continuously.

5. Method according to any of the preceding claims, wherein the natural processing aid or the processing aid derived from a natural substance is added to the seed yeast production step.

6. Method according to any of the preceding claims, wherein the natural processing aid or the processing aid derived from a natural substance is added to the commercial yeast production step.

7. Method according to either of Claims 5 and 6, wherein the natural processing aid or the processing aid derived from a natural substance is added discontinuously, preferably at the start of the respective production step.

8. Method according to Claim 5, wherein the production process of seed yeast is a fed-batch process and the concentration of the natural processing aid or the processing aid derived from a natural substance decreases in the course of said fed-batch process.

9. Method according to Claim 5, wherein the natural processing aid or the processing aid derived from a natural substance is added to the seed yeast washing medium.

10. Method according to any of the preceding claims, wherein the natural processing aid or the processing aid derived from a natural substance is selected from at least one of the group of hop acids comprising alpha acids, beta acids, rho-iso-α acids, iso-α acids, hexahydroiso-α acids, tetrahydro-iso-α acids and hexahydrobeta acids.

11. Method according to Claim 10, wherein tetrahydro-iso-α acids or beta acids are added as natural processing aid or the processing aid derived from a natural substance in the seed yeast production step.

12. Method according to Claim 11, wherein the final concentration of tetrahydro-iso-α acids is preferably in the range of 5 - 20 ppm, preferably 3 - 15 ppm, most preferably 2 - 10 ppm.

13. Method according to Claim 6, wherein beta acids are added as natural processing aid in the commercial yeast production process.

14. Method according to Claim 13, wherein the final concentration of beta acids is preferably in the range of 8 - 300 ppm, preferably 3 - 30 ppm, most preferably 1 - 12 ppm.

## Revendications

1. Procédé amélioré pour la production de levure, en particulier de levure de boulangerie, dans lequel on met la levure en contact avec un adjuvant de mise en oeuvre naturel ou un adjuvant de mise en oeuvre dérivé d'une substance naturelle, en une quantité suffisante pour supprimer ou au moins réduire l'activité de micro-organismes indésirables dans la production industrielle de levure d'ensemencement et/ou de levure commerciale, l'adjuvant de mise en oeuvre naturel ou l'adjuvant de mise en oeuvre dérivé d'une substance naturelle étant choisi parmi au moins un membre du groupe comprenant les acides de houblon ou un dérivé de ceux-ci.

2. Procédé selon la revendication 1 ou 2, dans lequel on ajoute à une source de glucide pour la culture de la levure l'adjuvant de mise en oeuvre naturel ou l'adjuvant de mise en oeuvre dérivé d'une substance naturelle avant l'addition de la culture de levure.

3. Procédé selon la revendication 2, dans lequel on ajoute l'adjuvant de mise en oeuvre naturel ou l'adjuvant de mise en oeuvre dérivé d'une substance naturelle avant ou après le traitement thermique du substrat glucidique pour la multiplication de la levure.

4. Procédé selon la revendication 2 ou 3, dans lequel on ajoute en continu l'adjuvant de mise en oeuvre naturel ou l'adjuvant de mise en oeuvre dérivé d'une substance naturelle.

5. Procédé selon l'une quelconque des revendications précédentes, dans lequel on ajoute l'adjuvant de mise en oeuvre naturel ou l'adjuvant de mise en oeuvre dérivé d'une substance naturelle dans l'étape de production de lavure d'ensemencement.

6. Procédé selon l'une quelconque des revendications précédentes, dans lequel on ajoute l'adjuvant de mise en oeuvre naturel ou l'adjuvant de mise en oeuvre dérivé d'une substance naturelle dans l'étape de production de levure commerciale.

7. Procédé selon l'une quelconque des revendications 5 et 6, dans lequel on ajoute en mode discontinu, de préférence au début de l'étape de production respective, l'adjuvant de mise en oeuvre naturel ou l'adjuvant de mise en oeuvre dérivé d'une substance naturelle.

8. Procédé selon la revendication 5, dans lequel pour ce qui est du procédé de production pour la levure d'ensemencement il s'agit d'un procédé discontinu avec additions et la concentration de l'adjuvant de mise en oeuvre naturel ou de l'adjuvant de mise en oeuvre dérivé d'une substance naturelle diminue au cours du processus discontinu avec additions.

9. Procédé selon la revendication 5, dans lequel on ajoute au milieu de lavage de la levure d'ensemencement l'adjuvant de mise en oeuvre naturel ou l'adjuvant de mise en oeuvre dérivé d'une substance naturelle.

10. Procédé selon l'une quelconque des revendications précédentes, dans lequel on choisit l'adjuvant de mise en oeuvre naturel ou l'adjuvant de mise en oeuvre dérivé d'une substance naturelle parmi au moins un membre du groupe des acides de houblon, qui comprend les acides alpha, acides bêta, acides rhoiso-α, acides iso-α, acides hexahydro-iso-α, acides tétrahydro-iso-α et acides hexahydro-bêta.

11. Procédé selon la revendication 10, dans lequel on ajoute dans l'étape de la production de levure d'ensemencement les acides tétrakhydro-iso-α ou les acides bêta en tant qu'adjuvant de mise en oeuvre naturel ou dérivé d'une substance naturelle.

12. Procédé selon la revendication 11, dans lequel la concentration finale d'acides tétrahydro-iso-α se situe de préférence dans la plage de 5-20 ppm, de préférence 3-15 ppm et de façon particulièrement préférée 2-10 ppm.

13. Procédé selon la revendication 6, dans lequel on ajoute des acides bêta en tant qu'adjuvant de mise en oeuvre naturel dans le processus pour la production de levure commerciale.

14. Procédé selon la revendication 13, dans lequel la concentration finale d'acides bêta se situe de préférence dans la plage de 8-300 ppm, de préférence 3-30 ppm et de façon particulièrement préférée 1-12 ppm.
